Europäisches Patentamt

European Patent Office

Office européen des brevets

(19)

(11) Veröffentlichungsnummer: **0 230 264 B1**

# EUROPÄISCHE PATENTSCHRIFT

(12)

(45) Veröffentlichungstag der Patentschrift: **22.09.93**

(51) Int. Cl.5: **A61K 47/02**, A61K 47/14

(21) Anmeldenummer: **87100419.8**

(22) Anmeldetag: **14.01.87**

(54) **Nasal applizierbares Arzneimittel, Verfahren zu seiner Herstellung und seine Verwendung.**

(30) Priorität: **23.01.86 DE 3601923**

(43) Veröffentlichungstag der Anmeldung:
**29.07.87 Patentblatt 87/31**

(45) Bekanntmachung des Hinweises auf die
Patenterteilung:
**22.09.93 Patentblatt 93/38**

(84) Benannte Vertragsstaaten:
**AT BE CH DE ES FR GB IT LI LU NL SE**

(56) Entgegenhaltungen:
**EP-A- 0 115 627**
**EP-A- 0 122 036**
**EP-A- 0 193 372**
**FR-A- 2 276 811**
**US-A- 4 226 848**

**Römpps Chemie-Lexikon, 8. Auflage, 1981, S.
1428**

(73) Patentinhaber: **BEHRINGWERKE Aktiengesellschaft**
**Postfach 1140**
**D-35001 Marburg(DE)**

(72) Erfinder: **Bremecker, Klaus-Dieter, Dr.**
**Höhenweg 78**
**D-3550 Marburg(DE)**
Erfinder: **Hungerer, Klaus-Dieter, Dr.**
**Oberer Eichweg 16**
**D-3550 Marburg(DE)**
Erfinder: **Ronneberger, Hansjörg, Dr.**
**Pappelweg 22**
**D-3550 Marburg(DE)**

## Beschreibung

Die vorliegende Erfindung betrifft Arzneimittel, die nasal verabreicht werden können und immunogene oder antigene Wirkstoffe in einem kolloid disperse Kieselsäure enthaltenden Gel enthalten, ein Verfahren zur Herstellung solcher Arzneimittel sowie ihre Anwendung.

Wirksame Vakzinen gehören zu den besten und preiswertesten vorbeugenden Mitteln gegen Infektionskrankheiten. Dabei haben sich besonders bakterielle Impfstoffe wie die gegen Tetanus, Diphtherie und Pertussis oder virale Impfstoffe wie die gegen Polio, Pocken und Masern bewährt. Neben der hohen Wirksamkeit vieler Präparate ist jedoch stets die Möglichkeit von Nebenwirkungen zu beachten. Die Ursachen solcher Nebenwirkungen sind häufig direkt mit der Wirkkomponente (zum Beispiel Lipopolysaccharide gramnegativer Bakterien), dem Adjuvans (zum Beispiel ölhaltige Präparationen) oder möglicherweise mit der Art der Applikation verbunden. Die meisten der in den letzten 80 Jahren entwickelten Impfstoffe müssen parenteral appliziert werden. Diese Applikationsart hat den großen Vorteil, daß jederzeit reproduzierbar eine genau definierte Dosis gegeben werden kann. Ein gewisser Nachteil könnten gewisse Nebenwirkung wie hyperergische oder neuroparalytische Reaktionen sein, die direkt mit der parenteralen Verabreichung in Zusammenhang stehen.

Aus diesem Grund wurden andere Applikationsarten als die parenterale untersucht. So kann heute gegen Typhus nach oraler Gabe des Impfstoffes ein Schutz aufgebaut werden. Auch eine orale, sublinguale Verabreichung von Tetanus-Toxoid ist beschrieben.

Als besonders interessant hat sich jedoch der Nasenraum zur topischen Applikation von Imfpstoffen und anderen pharmazeutisch wirksamen Präparaten erwiesen (US Patent 4,476,116). Die bekannten Präparate sind Nasentropfen oder Nasensprays. Diese galenischen Formen haben den Nachteil, daß eine exakte Dosierung der wirksamen Substanzmenge schwierig ist, da bei jeder Anwendung eine unbekannte Menge der Wirksubstanz über den Nasen-Rachen-Raum in den Verdauungstrakt gelangt und dort normalerweise nicht mehr die gewünschte Wirkung ausüben kann.

Es wurde überraschenderweise gefunden, daß Vakzinen und andere höhermolekulare pharmakologisch wirksame Präparate bei nasaler Verabreichung genau dosiert werden können, wenn die wäßrige Präparation durch Gelbildner gezielt in ihrer Fluidität eingeschränkt wird.

Gegenstand der Erfindung ist ein Arzneimittel zur nasalen Anwendung, dadurch gekennzeichnet, daß es einen höhermolekularen Wirkstoff, kolloid disperse Kieselsäure und Na-Dodecylsulfat, Na-Laurylsulfat oder Cetyl-stearoylsulfonsaures-Na enthält.

Ein solcher höhermolekularer Wirkstoff kann sein ein Immunogen, vorzugsweise ein bakterielles oder virales Immunogen oder eine pharmakologisch wirksame Substanz, vorzugsweise ein Hormon, besonders ein Peptidhormon.

Der Gelbildner enthält kolloid disperse Kieselsäure, beispielsweise ®Aerosil (Degussa, Frankfurt), in einer Konzentration von 1-15, vorzugsweise 2-8 g/100 g.

Das Gel wird stabilisiert durch Zusätze, vorzugsweise Na-Dodecylsulfat oder Na-Laurylsulfat, beispielsweise Texapon® L100 (Henkel, Düsseldorf) oder Cetyl-Stearoylsulfonsaures-Na wie z.B. Lanette® E (Henkel, Düsseldorf) in einer Konzentration von 0,05-15, vorzugsweise 0,1-8 g/100 g.

Als Löse- oder Quellmittel wird vorzugsweise Wasser eingesetzt. Dem Gel können Hilfsstoffe, beispielsweise Feuchthaltemittel wie Propylenglykol, Glyzerin, Sorbit oder Polyethylenglykol (PEG) zugesetzt werden. Zur Konservierung können nichtionische oder ionische Konservierungsmittel eingesetzt werden, vorzugsweise Propylenglykol, Na-Timerfonat, Sorbinsäure oder Benzoesäurederivate. Die Zubereitung kann mit bei der Salbenherstellung üblichen Maschinen und Geräten und unter entsprechenden Kautelen erfolgen.

Zur Schonung des einzuarbeitenden Wirkstoffes wird zunächst die Gelgrundlage nach in der pharmazeutischen Technologie üblichen Vorgehensweise hergestellt. In die vorgelegte Grundlage wird dann der Wirkstoff in gelöster, suspendierter oder trockener Form in geeigneter Konzentration eingearbeitet.

Mit einer solchen Formulierung kann die Wirksubstanz genügend genau auf die Nasenschleimhaut dosiert werden, wodurch die gewünschte Reaktion im Rahmen der biologischen Reproduzierbarkeit eingestellt werden kann. Sie kann nach Art einer Nasensalbe appliziert werden.

Die Herstellung einer solchen Formulierung entspricht der üblichen Salbenproduktion.

Die wirkstofffreie Grundlage wie auch die fertige Zubereitung wird auch nach mehrfacher Applikation von der Schleimhaut ohne erkennbare Reizung vertragen. Am Beispiel von Tetanus- und Diphtherie-Toxoid als Wirkstoff wurde gezeigt, daß im Vergleich zu konventionellen nasalen Zubereitungen (Lösung, Spray, Salbe) die Bioverfügbarkeit optimiert und die Streuung der Titer minimiert ist.

Das beschriebene Trägersystem eignete sich zur Inkorporierung von niedermolekularen Wirkstoffen wie von Makromolekülen, besonders von Toxoiden wie denen von Tetanus, Staph. aureus, Diphtherie oder von Bakterienextrakten wie solchen von Pseudomonas oder von Hormonen, besonders von Peptidhormonen wie Insulin oder von Antikoagulantien wie Liquemin® oder von Immunglobulinen, die polyklonale oder monoklonale Antikörper enthalten.

Die erfindungsgemäßen Arzneimittel können zur Prophylaxe oder Therapie von Krankheiten verwendet werden.

Diese Arzneimittel können am Menschen oder am Tier angewendet werden.

Im Folgenden wird beispielhaft die Herstellung einiger Formulierungen beschrieben.

<u>Beispiel 1</u>

```
Aerosil® 200                          0,5 g
Lanette® E                            0,5 g
Tetanustoxid (TT; 2000 LF*/ml)        2,5 ml
Diphtherietoxid (DT; 2000 LF/ml)      2,5 ml
Wasser ad                            10,0 g
```

Aerosil® wurde in der wässrigen Lösung von Lanette® E unter Gelbildung suspendiert. Die beiden wirkstoffhaltigen Lösungen wurden eingearbeitet.
0,2 g der Zubereitung entsprachen 100 LF TT und DT.

*) LF = Limes flocculationis (WHO Requirements, Technical Report Series (1964), 293)

Lanette® N, Propylenglykol, Karion® F und Wasser wurden unter Erwärmen homogenisiert. In die erhaltene Grundlage wurden die Toxoide eingearbeitet.

Die Zubereitungen der folgenden Beispiele wurden entsprechend Beispiel 1 hergestellt.

| Beispiel 2 | |
|---|---|
| Aerosil® 200 | 0,6 g |
| Texapon® L100 (10 % wäßrige Lösung) | 5,4 g |
| Staph. aureus alpha-Toxin-Toxoid (600 BE) | 1,0 ml |

| Beispiel 3 | |
|---|---|
| Aerosil® 200 | 0,24 g |
| Lanette® E (10% wäßrige Lösung) | 5,4 g |
| Pseudomonas Extrakt hergestellt nach Behring Institute Mitteilungen, Nr. 76, 113-120 (1984); "Vaccine production" | 0,2 g |
| Wasser ad | 4,8 g |

EP 0 230 264 B1

| Beispiel 4 | |
|---|---|
| Aerosil® 200 | 0,4 g |
| Lanette® E (10% wäßrige Lösung) | 3,6 g |
| Insulin CR (Hoechst AG) | 2 g |
| Wasser | 2 g |

| Beispiel 5 | |
|---|---|
| Aerosil® 200 | 0,5 g |
| Lanette® E (10% wäßrige Lösung) | 4,5 g |
| Liquemin® 2500 (Natriumheparinat; Hoffmann-La Roche) | 4 ml |
| Wasser ad | 10 g |

**Patentansprüche**

1. Arzneimittel zur nasalen Anwendung, dadurch gekennzeichnet, daß es einen höhermolekularen Wirkstoff, kolloid disperse Kieselsäure und Na-Dodecylsulfat, Na-Laurylsulfat oder Cetyl-stearoylsulfonssaures-Na enthält.

2. Arzneimittel nach Anspruch 1, dadurch gekennzeichnet, daß der Wirkstoff ein Immunogen oder eine andere pharmakologisch wirksame Substanz, vorzugsweise ein bakterielles oder virales Antigen, oder ein Hormon ist.

3. Arzneimittel nach Anspruch 1, dadurch gekennzeichnet, daß es die Kieselsäure in einer Konzentration von 1-15 g/100 g enthält.

4. Arzneimittel nach Anspruch 1, dadurch gekennzeichnet, daß es ein Feuchthaltemittel und/oder Konservierungsmittel enthält.

5. Verfahren zur Herstellung eines Arzneimittels nach Anspruch 1, dadurch gekennzeichnet, daß die Kieselsäure-Grundlage hergestellt und der Wirkstoff in gelöster, suspendierter oder trockener Form in wirksamer Konzentration eingearbeitet wird.

**Claims**

1. A pharmaceutical for nasal administration, which contains a high molecular weight active compound, colloidal disperse silica and Na dodecyl sulfate, Na lauryl sulfate or Na cetylstearoylsulfonate.

2. A pharmaceutical as claimed in claim 1, wherein the active compound is an immunogen or another pharmacologically effective substance, preferably a bacterial or viral antigen, or a hormone.

3. A pharmaceutical as claimed in claim 1, which contains the silica in a concentration of 1-15 g/100 g.

4. A pharmaceutical as claimed in claim 1, which contains a humectant and/or preservative.

5. A process for the preparation of a pharmaceutical as claimed in claim 1, which comprises preparing the silica base and incorporating the active compound in dissolved, suspended or dry form and in effective concentration.

**Revendications**

1. Médicament pour l'application nasale, caractérisé en ce qu'il contient une substance active à masse moléculaire élevée, de la silice en dispersion colloïdale et du dodécylsulfate de Na, du laurylsulfate de Na ou du cétyl-stéaroylsulfonate de Na.

4

2. Médicament selon la revendication 1, caractérisé en ce que la substance active est un immunogène ou une autre substance pharmacologiquement active, de préférence un antigène bactérien ou viral, ou une hormone.

3. Médicament selon la revendication 1, caractérisé en ce qu'il contient la silice à une concentration de 1 - 15 g/100 g.

4. Médicament selon la revendication 1, caractérisé en ce qu'il contient un agent de maintien de l'humidité et/ou un conservateur.

5. Procédé pour la fabrication d'un médicament selon la revendication 1, caractérisé en ce que l'on prépare l'excipient à base de silice et on y incorpore la substance active à l'état sec, dissous ou en suspension, à une concentration efficace.